# EUROPEAN PATENT SPECIFICATION

(11) **EP 0 418 301 B1**
(45) Date of publication and mention of the grant of the patent: **19.10.1994**
(21) Application number: 89906842.3
(22) Date of filing: 12.06.1989
(51) Int. Cl.: A61F 2/32

(54) **HIP JOINT PROSTHESIS**
PROTHESE FÜR HÜFTGELENK
PROTHESE D'ARTICULATION DE LA HANCHE

(30) Priority: 10.06.1988 SE 8802194
(43) Date of publication of application: 27.03.1991
(73) Proprietor: Astra Aktiebolag, 151 85 Södertälje (SE)
(72) Inventor: Albrektsson, Björn, S-431 38 Mölndal (SE); Carlsson, Lars, S-430 41 Kullavik (SE); Wennberg, Stig, S-424 57 Angered (SE); Rostlund, Tord, S-430 41 Kullavik (SE)
(86) International application number: SE8900329
(87) International publication number: WO8911837

(56) References cited:
- DE-A- 2 724 234
- DE-A- 2 845 231
- FR-A- 2 344 272
- US-A- 2 612 159

## Description

### Technical field:

The present invention relates to a hip joint prosthesis for permanent anchoring in the human hip joint, which prosthesis consists of a first joint unit designed to be anchored in the neck of a human femur and a second joint unit designed to be anchored in the human pelvis, in which respect the first joint unit comprises a joint head and an anchoring arrangement for anchoring the joint head in the femur, and the second joint unit comprises a socket with a concave joint surface shaped and dimensioned to bear against and to articulate under sliding contact with the joint head, and an anchoring arrangement for anchoring the socket in a cavity in the pelvis, the anchoring arrangement in the femur comprising at least one sleeve-shaped anchoring element and at least one tightening element connected to the joint head in order to create, by engaging with the anchoring element, a prestressable anchoring of the joint head with the tightening element introduced into the sleeve-shaped anchoring element, the tightening element comprising a head and being designed to be axially introduced into the associated sleeve-shaped anchoring element and connected to the joint head. Such a hip joint prosthesis is known from the document DE-A-28 45 231.

### Prior art:

On the market and in clinical use there are at present a number of different hip prosthesis designs which all have the common feature that the prosthesis components are anchored in connective tissue. This applies, on the one hand, to prostheses intended for anchoring by means of bone cement and, on the other hand, prostheses intended for cement-free anchoring by screwing into the bone tissue or by creating primary fixation by means of a positive lock in another way, and at the same time creating the preconditions for further consolidation by bone tissue growing into the porous surface structures of the associated prosthesis components. The latter principle, which thus involves first creating a positive lock on the macroplane between bone and prosthesis and secondarily creating a positive lock on the microplane by osseous in-growth, has led to extensive marketing by a number of different companies which have presented variations on the same basic theme, even if irregularities in the prosthesis surface have been produced by different technical methods. The forerunner in this field is the Lord prosthesis which it has been shown can be fixed very satisfactorily to bone tissue, but without direct contact between prosthesis component and bone tissue, and, above all, without the necessary physiological load interplay between prosthesis and bone tissue. This has meant that the bone tissue has disappeared in places on account of the fact that it has not been able to transmit physiological load. This problem has been reduced in prosthesis components produced in later years, but there is still no convincing proof that today's prosthesis designs for cement-free anchoring in the hip joint are superior to yesterday's joint replacements in the form of cemented prostheses.

The basic design which has predominated, regardless of the anchoring principle, has involved the acetabulum being replaced by a joint socket of high-density polyethylene which has either been cemented or screwed into the bone base with or without prestressing, and at the same time the joint ball has been replaced by a metal ball provided with a stem which goes down into the medullary cavity of the hip bone. The stem length has varied between 10 and 30 cm. The stems of the cement-free prosthesis systems have had a surface structure for osseous in-growth either along the entire stem or along only parts thereof.

There have been departures from the abovementioned basic design, which have characterized a number of different hip prostheses. One example of these is the Ring prosthesis whose acetabulum was screwed securely with a long stem screw up into the pelvis. Another exception is the double-cup prosthesis (Wagner and ICLH) in which the joint ball consisted of a stemless hood covering the femoral neck, which was preserved to the greatest possible extent using this type of prosthesis. The usual prostheses provided with a stem, of the Charnley, Müller or McKee-Ferrar type, assumed that the greater part of the femoral neck was removed in connection with the prosthetic surgery. Patent publication DE-A-2,845,231 discloses a prosthesis in which respect it is assumed that anchoring of the joint ball unit is obtained by direct osseous in-growth in porous surface structures. The known prosthesis has a non-threaded anchoring sleeve which is so short that the main part of a shaft incorporated in the joint ball unit makes bone contact, and this means that joint surface replacement must be effected in a single stage with simultaneous assembly of both an anchoring arrangement and the joint ball unit.

### Technical problem:

At present it may be said that in Sweden it has been possible for about 20 years now to replace the hip joint with good results, but not without risks of complications, which increase with time after introduction of the prosthesis system. In patients with degenerative disorders or rheumatism, the anticipated benefits of hip prosthesis surgery are generally so great that an operation is recommended without hesitation despite awareness of the risks of complications such as infection and, above all, mechanical loosening. The risk of mechanical loosening increases with time after the operation and with the level of activity of the patient. The latter risk factor has limited the indication to essentially include patients of 60 years and above. In these cases a lower level of activity and a shorter remaining life span are expected. In the case of younger patients the attitude to prosthetic surgery is one of hesitation, as a result of doubts regarding the reliability of the joint replacements on offer today and doubts regarding, first, the anchoring stability and, second, the risks related to the body's reaction to foreign material incorporated in the prosthesis. It is clear that there is a considerable requirement among patients of less than 60 years of age for hip prostheses with better guarantees in respect of anchoring stability and biocompatibility.

Since it has been possible in Sweden for more than 20 years now to follow up hip prostheses anchored with cement and, for less than 10 years, to follow up prostheses anchored without cement, it has been observed that there is an increased incidence of mechanical loosening with time, regardless of the choice of anchoring method. It has been possible to demonstrate histologically that no more than a point-wise direct contact has been achieved between bone tissue and prosthesis component, which does not rule out the possibility that the prosthesis component may have been anchored very firmly in the bone base via fibrous tissue encapsulating the prosthesis. Against the background of this experience, it is interesting to note that there is a Swedish method for skeletal anchoring of dental bridges with dental implants in which the anchoring stability, on the one hand, far exceeds that obtained earlier after joint replacement by the abovementioned methods and, on the other hand, the initially achieved anchoring stability is maintained for over 20 years after the skeletal implantation. This unique anchoring stability of a skeletal implant is probably dependent on the creation of a direct contact free from connective tissue between the bone tissue and implant, so-called osseointegration. The preconditions for this type of unique anchoring have been shown to be the use of commercially pure titanium as metal component with unique biocompatibility and corrosion resistance, combined with a controlled and atraumatic operating technique, and the avoidance of direct loading by means of the implant being inserted in a 2-stage procedure in which an anchoring element is simply implanted in the first stage and is only coupled to a loaded function element in a second operation.

One aim of the present invention is to provide a hip joint prosthesis which is based on the experiences from the abovementioned Swedish method for jaw implants. The aim is, therefore, to create an osseointegrated hip joint prosthesis in which the metal component consists of titanium and in which anchoring element and function element are introduced in separate operating stages with a load-free interval of at least 3 months. In the present invention it is of course intended, in the first place, to create the conditions for a satisfactory anchoring stability which will make it possible to extend the indications for hip prosthesis surgery to more active and younger individuals than has been the case up till now, but a further aim is to reduce the theoretical risks related to the transport of toxic metal ions from the prosthesis material to the body tissues. Although the metal alloys used in prosthesis systems in clinical use today offer a good compromise as regards satisfying requirements for mechanical strength, wear resistance, corrosion resistance and production economy, important objections can theoretically be raised as regards their biocompatibility properties. The alloy substances, such as chromium, cobalt, nickel, aluminium and vanadium, are thus potentially toxic, even though it has not been possible to elucidate the threshold value for toxic and allergic reactions to these metals. For example, in the world today there are only 10 or so reported cases in which there has been cause to suspect a connection between development of cancer and the use of prostheses of cobalt-based alloy, but there is nevertheless concern that this type and other types of complications are essentially increasing in parallel with the increasing use of prosthesis systems for the large joints of the body. As regards commercially pure titanium, no case has as yet been described where it was possible to report either allergy or any other toxic reaction to the metal in question. Both experimental studies and clinical experience indicate that commercially pure titanium is superior to alternative implant metals from the point of view of biocompatibility.

One aim of the present invention is to produce a hip joint prosthesis by means of which the surgical defect in prosthetic surgery is reduced, i.e. the cutting of intact bone tissue in connection with the operation can be reduced by preserving a large part of the femoral neck.

### Technical solution:

The said aims are achieved by means of a hip joint prosthesis according to the present invention, which is characterized in that the sleeve-shaped anchoring element has a grooved outer surface and is designed to be separately implanted in a channel passing through the femoral neck and to extend essentially over the entire length thereof in order to form an anchorage in the femur via the said outer surface and to form a guide for the tightening element of the first joint unit and an axle guide for a cutting instrument rotatable about an axle for shaping at least one bearing surface of the femoral neck, extending rotationally symmetrical about the longitudinal axle of the said tightening element, to the shape of at least one corresponding bearing surface of the joint head extending rotationally symmetrical about the attachment of the said tightening element in the joint head.

### Description of figures:

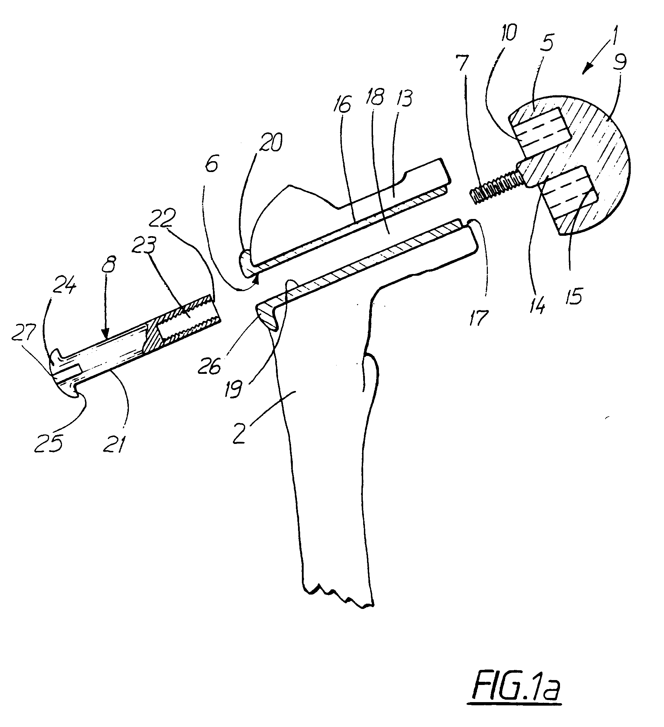

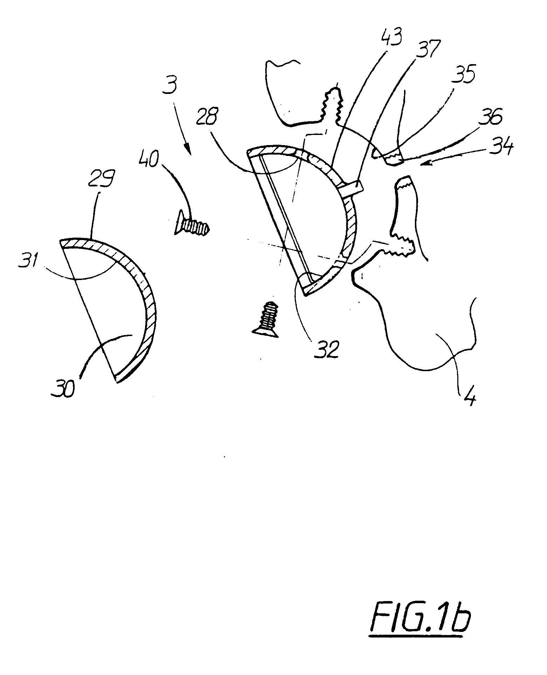

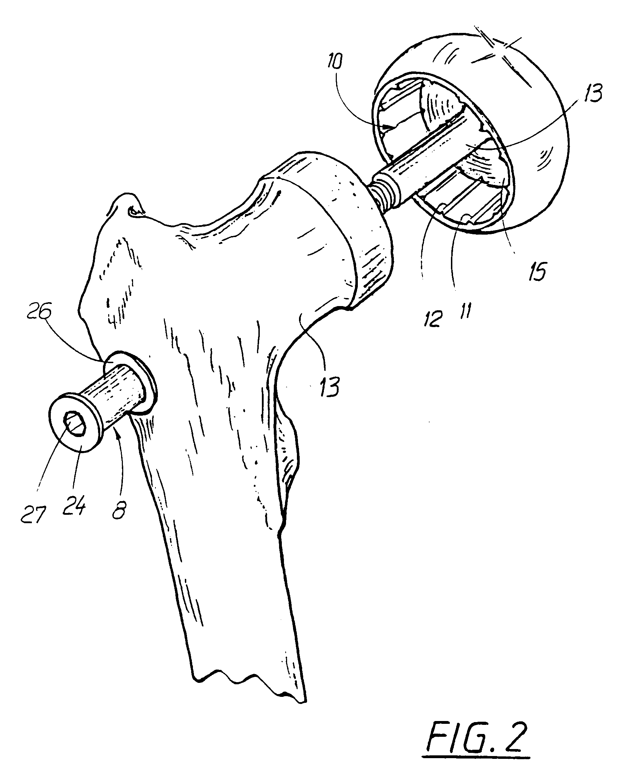

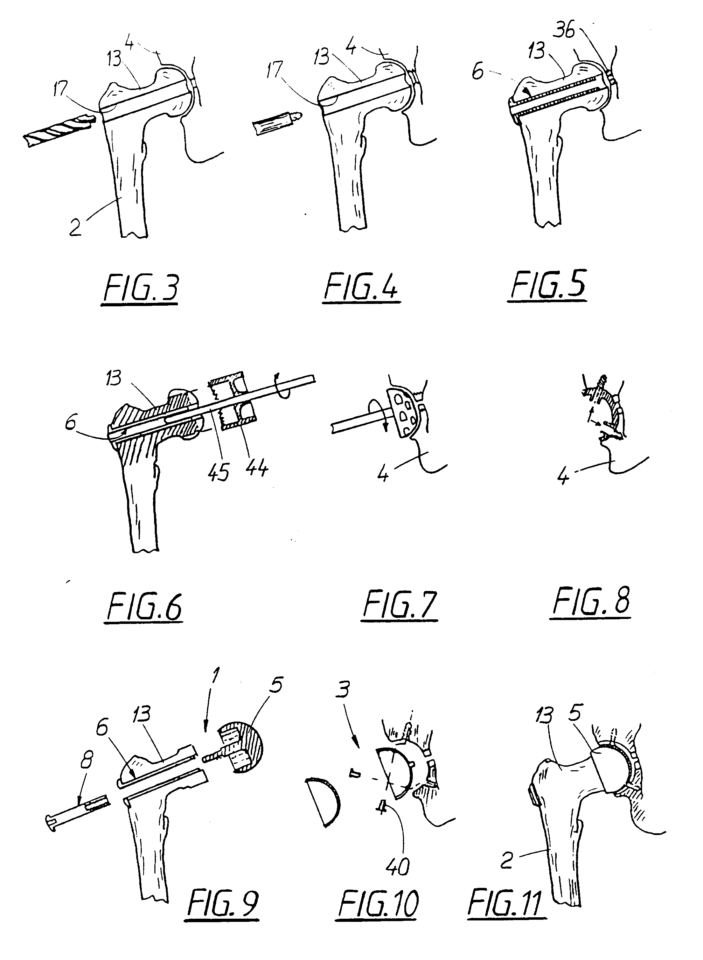

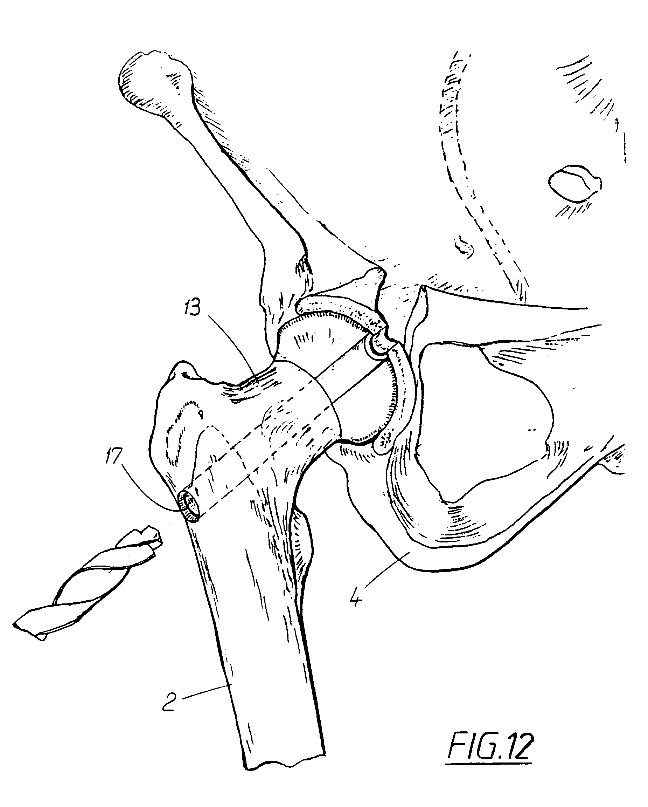

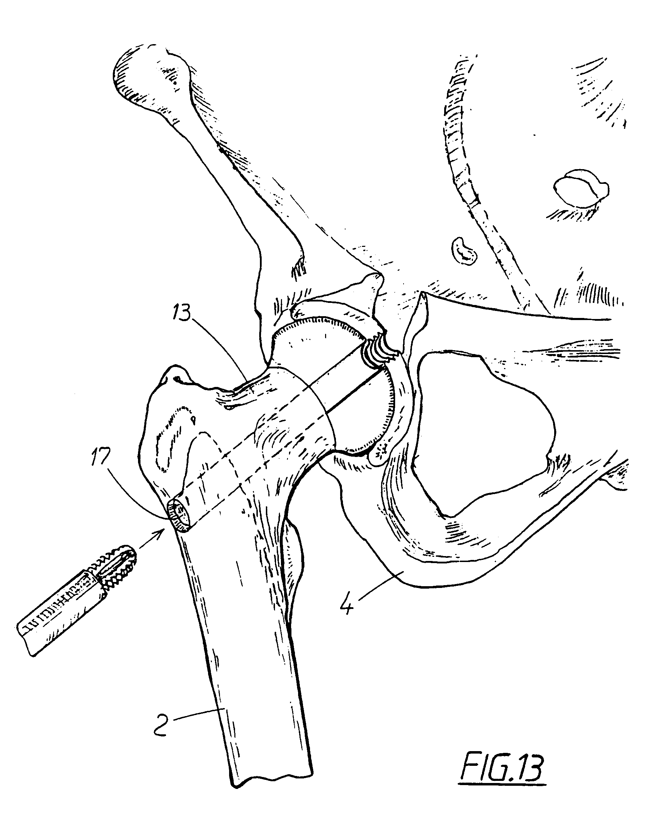

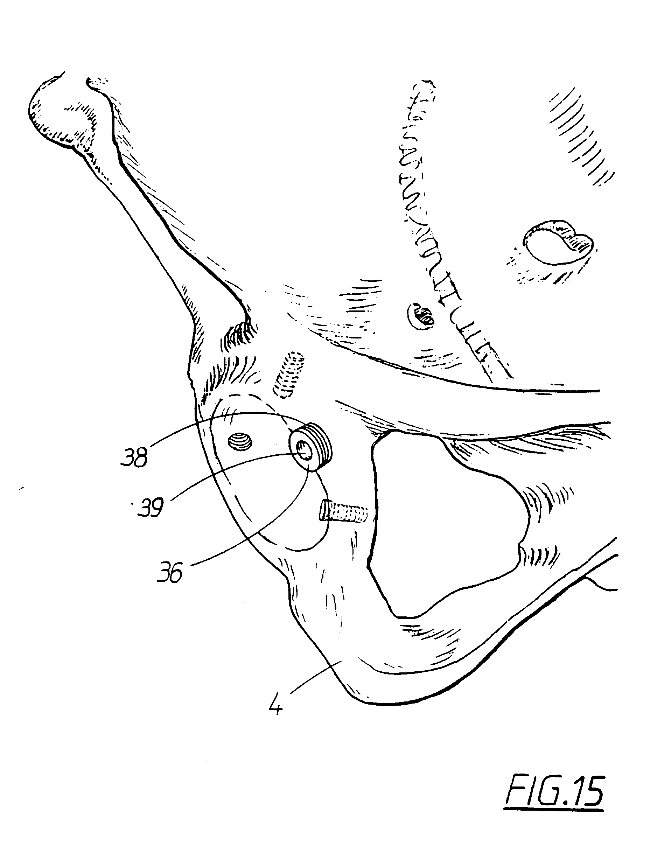

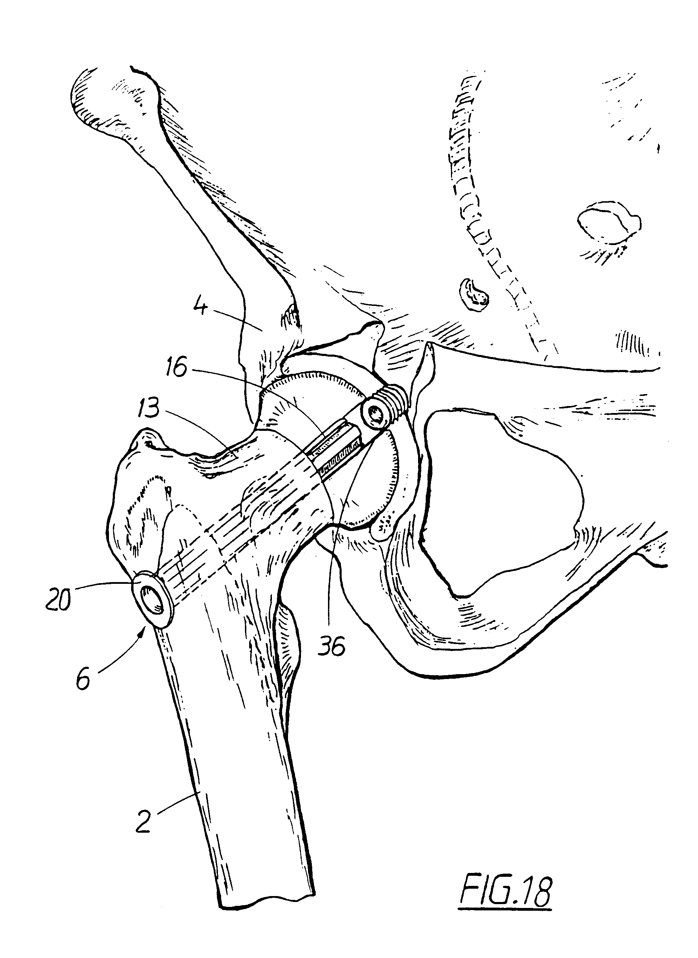

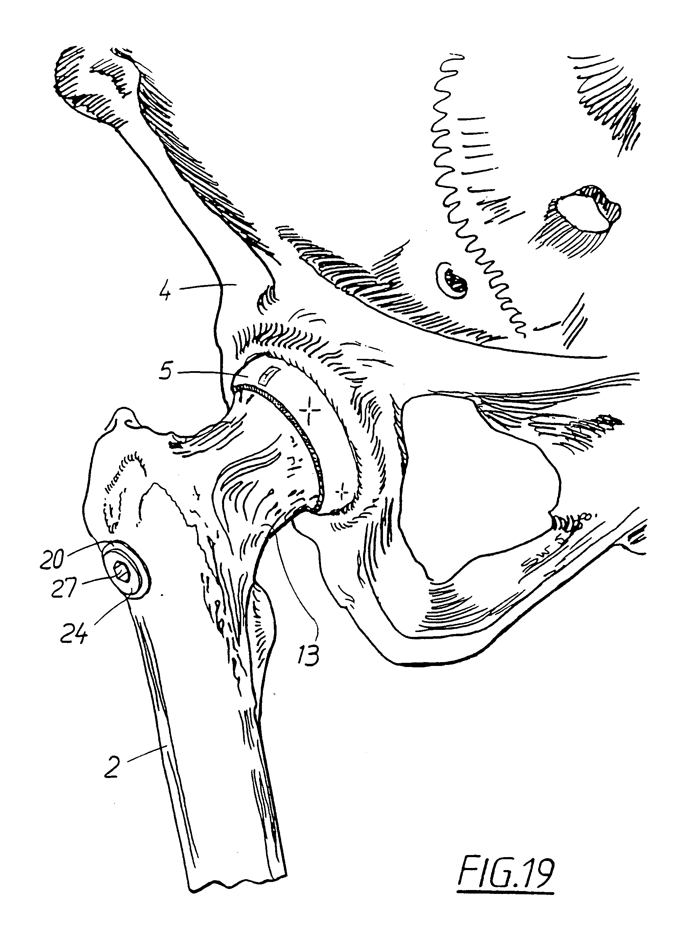

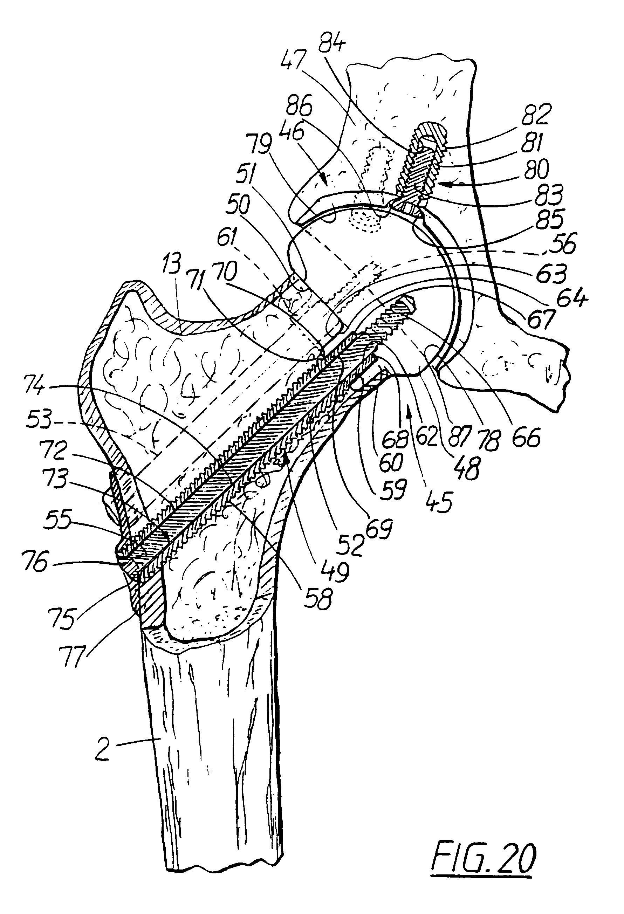

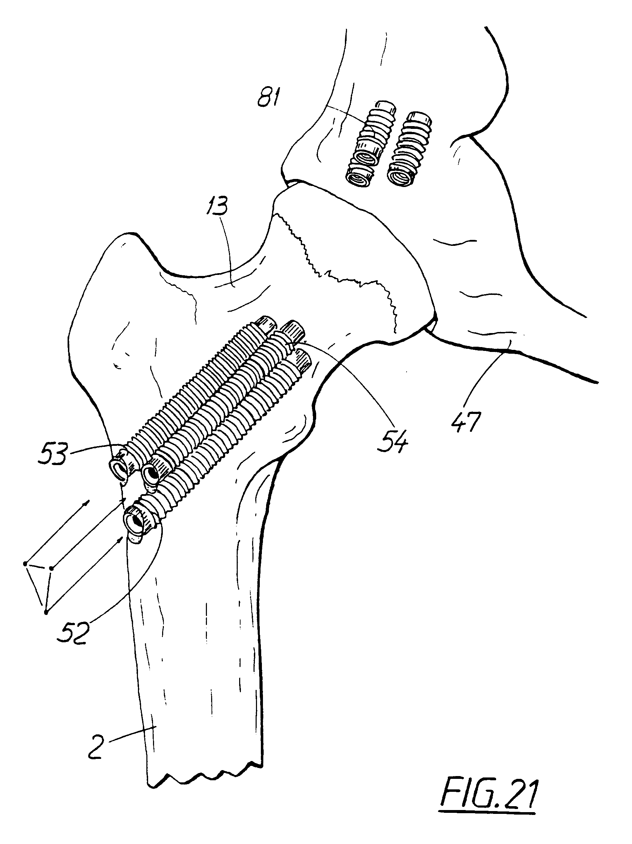

The invention will be described in greater detail below on the basis of a number of exemplary embodiments and with reference to the attached drawings in which Figs. 1a and 1b show exploded views in section of the components incorporated in a first embodiment of the hip joint prosthesis according to the present invention. Fig. 2 shows a perspective view of the joint ball unit incorporated in the prosthesis. Figs. 3 - 11 show schematically the procedure for assembly of the hip joint prosthesis according to Figs. 1a and 1b, while Figs. 12 - 18 show, on a larger scale, partially broken perspective views of the assembly procedure in greater detail, and Fig. 19 shows a hip joint provided with the hip joint prosthesis in the first embodiment according to the present invention. Fig. 20 shows the hip joint prosthesis in a second embodiment. Figs. 21 - 24 show the different stages in the implantation of the prosthesis according to Fig. 20, and Figs. 25 and 26 show two further embodiments of the prosthesis according to the invention.

### Preferred embodiments:

The design of the hip joint prosthesis in the first embodiment emerges most clearly from Fig. 1a, Fig. 2 and Fig. 16. The prosthesis consists of two main units - on the one hand a ball unit 1 intended to be anchored in a subject's femur 2 and, on the other hand, a socket unit 3 intended to be anchored in a subject's pelvis 4 - in order to replace degenerated or damaged joint surfaces in a subject's hip joint. The ball unit 1 consists of a ball 5 and an anchoring arrangement, comprising an anchoring element 6 and a screw connection in the form of a threaded pin 7 projecting out from the ball 5 and a threaded screw bushing 8.

The ball 5 is made, for example, of titanium and has a convex joint surface 9 which has the shape of a partial sphere, essentially an approximated semisphere with an essentially cylindrical cavity 10, which is open in the direction away from the joint surface 9. As emerges most clearly from Fig. 2, the cylindrical inner wall 11 of the cylindrical cavity 10 is designed with profiling, in the illustrated example in the form of ribs 12 in order to permit secure positive locking against the femoral neck 13 with deformation of its surface. In the example shown, the ribs extend in the axial direction parallel to the longitudinal direction of the threaded pin 7, but they can also have another design such as, for example, threads for engaging with the femoral neck. The pin 7 extends centrally and axially relative to the cylindrical cavity 10 and forms part of a shaft 14 which extends centrally and axially and projects from the base 15 of the cavity 10. The unthreaded part of the shaft 14 is essentially cylindrical and has a diameter which exceeds the diameter of the threaded pin 7, and its shape and dimensions are adapted to the shape and dimensions of the anchoring element 6 in such a way that the shaft 14 can be introduced into the anchoring element.

The anchoring element 6 forms, as shown most clearly in Fig. 1a, a femoral neck fixture in the form of a sleeve of, for example, titanium with a grooved, for example threaded, contact surface against the bone. This sleeve is essentially cylindrical and has an outer wall 16, for example cylindrical, intended for anchoring in a drilled channel 17 through the femoral neck 13, and a continuous cylindrical channel 18 with a cylindrical inner wall 19 dimensioned so as to form a guide for the shaft 14 of the ball 5. The anchoring element 6 has, at its end distant from the ball 5, a flange 20 intended to bear against the femur 2 in an area around the outer end of the channel 17. The outer wall 16 of the anchoring element 6 can be profiled by means of elongate ribs or threads for good anchoring in the femoral neck.

The screw bushing 8 is designed as an essentially cylindrical body with a cylindrical outer wall 21, the shape and dimensions being adapted to be introduced into the channel 18 of the anchoring element 6 so as to be positionally guided in the latter with good precision. The cylindrical wall 21 of the screw bushing is expediently given the same diameter as the shaft 14. The screw bushing 8 also has a central bore 23 open at one end 22 with an internal thread dimensioned to cooperate with the screw 7 of the ball 5 in order to form the screw connection. The screw bushing 8 furthermore has a head 24 which comprises a stop surface 25 which forms an abutment against a bearing surface 26 on the flange 20 of the anchoring element 6. In the example shown, the head is designed as a hexagonal screw with a hexagonal recess 27 for tightening the ball 5 with a hexagonal wrench and prestressing it, as will be described in greater detail hereinbelow. Another design of the head, for example with an external polygonal form, helical grooves or the like, is of course conceivable.

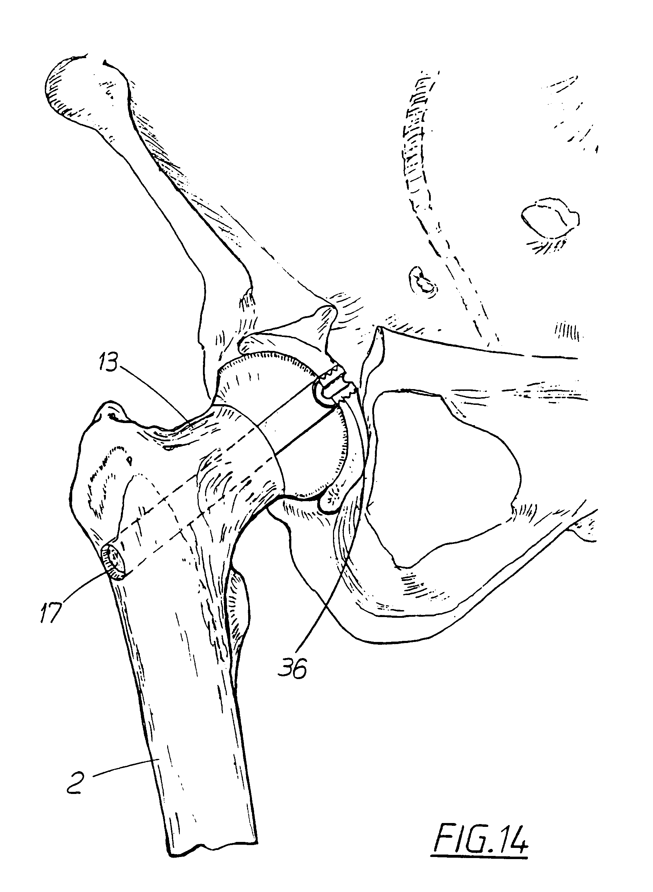

In the example shown, the socket unit 3 is designed as a double socket with a load-absorbing base socket 28 made of a strong, tissue-compatible material, such as titanium, and a socket made of a material which gives low friction against the ball 5, for example a wear-resistant polymer material such as high-density polyethylene. The socket has a concave and essentially semispherical cavity 30 whose form and dimensions are adapted to the external form and dimensions of the joint surface 9 of the ball 5. The concave wall 31 of the socket 29 thus forms the joint surface of the socket unit 3. The socket 29 has a convex and essentially semispherical fitting surface 31 intended to be introduced into a correspondingly shaped and dimensioned and essentially semispherical concave bearing surface 32 in the base socket 28. The socket 29 is intended to be connected to the base socket 28 by means of a positive lock which, in the example illustrated in Fig. 14, consists of a snap lock in the form of a circumferential rib 33 on the fitting surface 31 of the bearing socket, which rib 33 is intended to snap into a corresponding groove 34 in the bearing surface of the base socket 28.

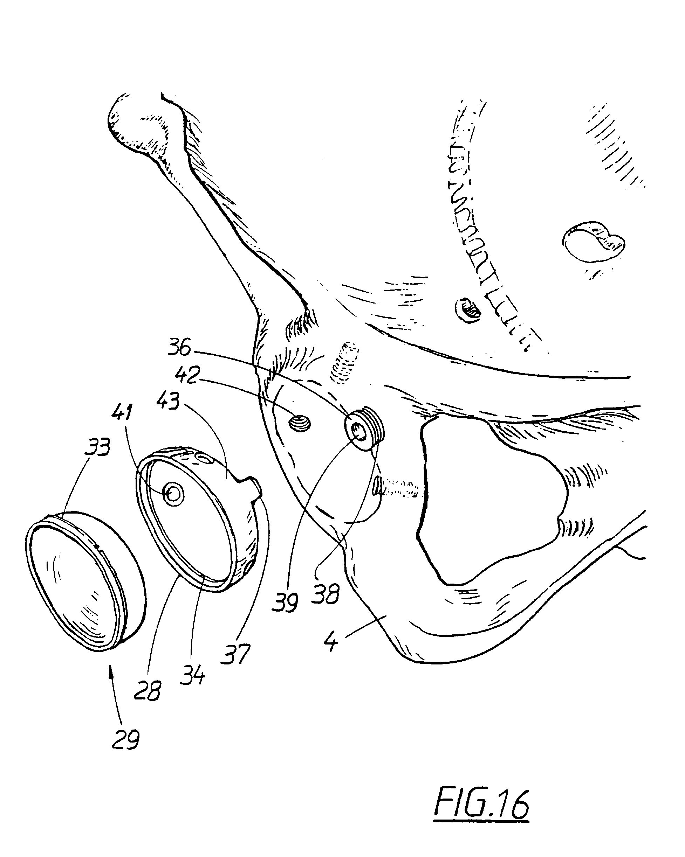

The socket unit 3 (see Fig. 16) moreover comprises an anchoring arrangement 34 for anchoring the base socket 28 in the hollow 35 of the pelvis 4 adapted by pre-forming to the shape of the base socket 28. The anchoring arrangement comprises at least one anchoring element 36 in the form of a pelvis fixture, for example of titanium, comprising a positive lock for cooperation with a central pin 37 which projects from the base socket 28. As described in greater detail below, the anchoring element 36 of the pelvis 4 is, in a first operation, anchored in a recess formed in the pelvis and has an external profile 38 which, by means of deformation of the walls in this recess, ensures a positive engagement and a growing together with the bone tissue. As in the example shown, this profile can be a screw thread or axially extending grooves. On the inside the anchoring element 38 has a threaded bore 39 and is therefore in the form of a sleeve or bushing into which the base socket 28 can be introduced by means of its tapered pin 37. Moreover, in the example shown, the anchoring arrangement 34 has a number of anchoring screws 40 (see Fig. 1b) with countersunk heads intended to be introduced into bevelled holes 41 in the base socket 28. The anchoring screws 40 are intended to be anchored in bores 42 formed in the hollow 35 of the pelvis 4. The inside 43 of the base socket 28 is, like the other main surfaces of the socket unit 3, essentially semispherical and forms a fitting surface for load-absorbing support against the concave hollow 35 in the pelvis.

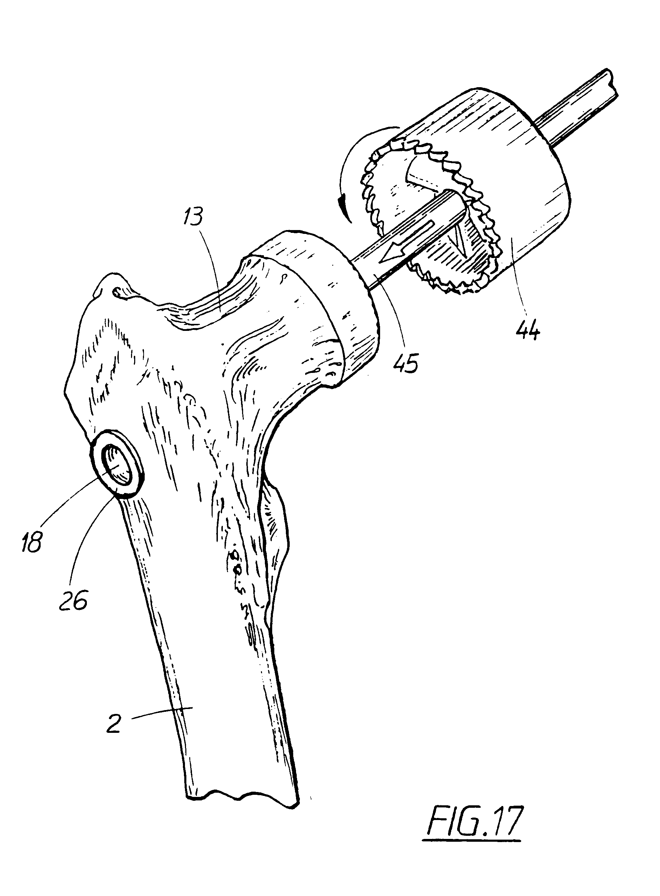

Before the ball is screwed onto the femoral neck and the central shaft 14 slides into the femoral neck fixture 16, a trepan 44 is used (see Figs. 6 and 17) to adapt the dimension of the femoral neck 13 so that it affords a positive lock when the ball 5 with its cutting profile is tightened or screwed on. So that both the threaded cylinder wall of the ball and the central shaft are adapted correctly on the one hand to the outer contour of the femoral neck and, on the other hand, to its fixture, the trepan for form adaptation of the femoral neck is fixed with a central guide pin 45 which goes into the fixture which in this respect forms a guide bearing. The rotation of the trepan about the guide pin 45 produces a bearing surface, rotationally symmetrical relative to the longitudinal axis of the anchoring element, on the femoral neck whose shape is adapted to the cylindrical inner wall 11 of the joint head. There are, for example, three trepans with different internal diameters corresponding to three differently dimensioned joint balls intended for femoral necks of three different dimensions.

When the titanium ball is screwed securely onto the femoral neck, the central shaft 14 with both its cylindrical and threaded part slides into the centre of the cavity in the femoral neck fixture, which cavity constitutes a space for both the pin and also the internally threaded locking sleeve introduced from the other end of the fixture. This sleeve is provided with a prestressing device against the fixture so that, when it is threaded against the ball pin, it causes the ball to press against the femoral neck. Thus, as a result of the adaptation of its form to the neck and as a result of the screw connection of the ball shaft against the compression sleeve, the ball acquires, on the one hand, a positive locking and, on the other hand, a prestressing against its bone base.

Before the ball in the second operation has been screwed onto the femoral neck, the titanium socket is fixed in the acetabulum by means of, on the one hand, the central outer pin 37 of the socket, which pin forms a male connection in the female seat of the pelvic fixture, and, on the other hand, screw connections directly to the bone tissue via the holes 41 in the titanium socket. The pin on the socket has a conical finish towards its socket attachment with a gradient of 1:15. This compensates for tensile stress at the socket-leg transition, but the primary function of the pin is to neutralize the moment which arises via the various compression screws 40 which risk causing both tensile and shearing stress on the opposite side of the socket-leg connection. In order to reduce such risk-related moments, it is desirable to increase the contact pressure between metal socket and bone optimally by prestressing by means of compression screwing. However, this presupposes that the contour of the socket follows that of the bone seat, and this is facilitated by bone transplantation between socket and pelvis. The titanium socket is thus fitted on a bone surface, well adapted by bone transplantation, by means of, on the one hand, the central conical pin and, on the other hand, three to five compression screws introduced through holes in the titanium socket. Thereafter, the joint socket is fitted to the base socket via the snap attachment 33, 34. The joint socket, which consists for example of high-density polyethylene, is now fixed to the acetabulum via, on the one hand, an osseointegrated anchoring element in the form of a titanium fixture and, on the other hand, the base socket with its compression screws.

The two operations required for introducing the hip joint replacement should be separated by an interval of at least 3 months. The first surgical intervention can be carried out via a small lateral incision, such as is used in nailing of a fracture of the collum, and in this operation, under fluoroscopy and with the patient on one side, a central hole is made in the head of the collum and this hole is extended into the acetabular base of the pelvis.

The procedure is carried out in a precise manner, as in collum nailing, starting with a Kirschner wire in order to find the ideal position with the aid of TV fluoroscopy, and then a cannulated drill and a cannulated thread tap are used to make a hole through the entire head and the acetabular base with a diameter of about 11 to 12 mm. The pelvic fixture which is very short is then introduced by means of a long insertion device through the hole in the collum head, after which the femoral neck fixture is introduced through the same hole up to a level with the cartilage-bone limit on the head.

The second operation requires considerably greater intervention. The approach must be from the front with regard to the anteversion angle of the collum, and the incision and technique are approximately the same as used for the ICLH prosthesis. As in the introduction of a double cup, a trepan is used to cut the head of the collum, which trepan has been described above and can advantageously be manually operated. As described above, the trepan has a central pin which is introduced into the femoral neck fixture, and the trepan is guided so as to cut the bone. This can be carried out in several stages if the bone is hard and, in this case, a series of trepans of gradually decreasing diameters is used.

Once the hip joint surface replacement has been introduced in the second operation, a situation exists in which there are well anchored joint surface replacements which, by means of good contact adaptation and prestressing, permit early exercising, but with a primary load reduction.

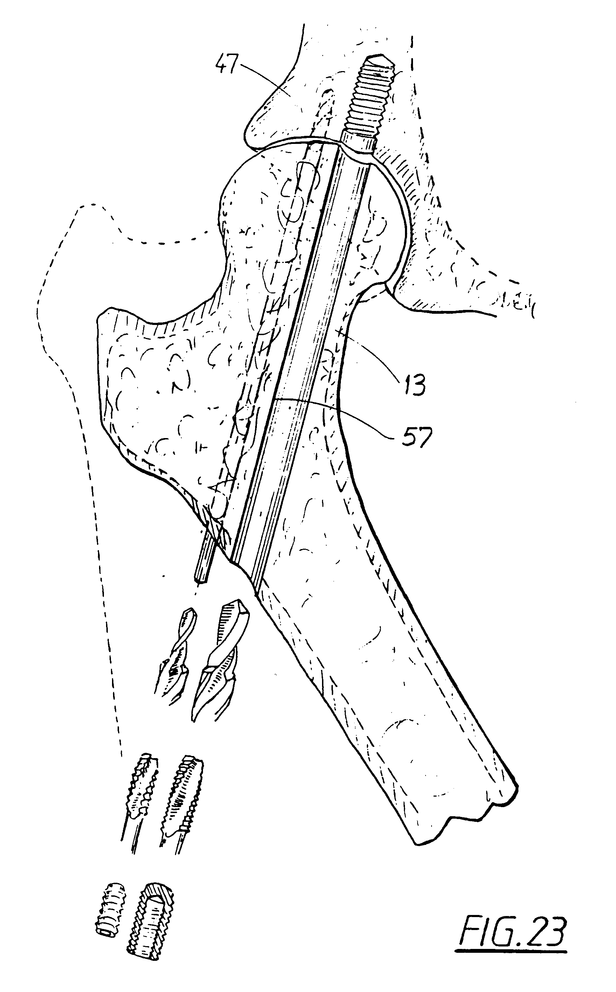

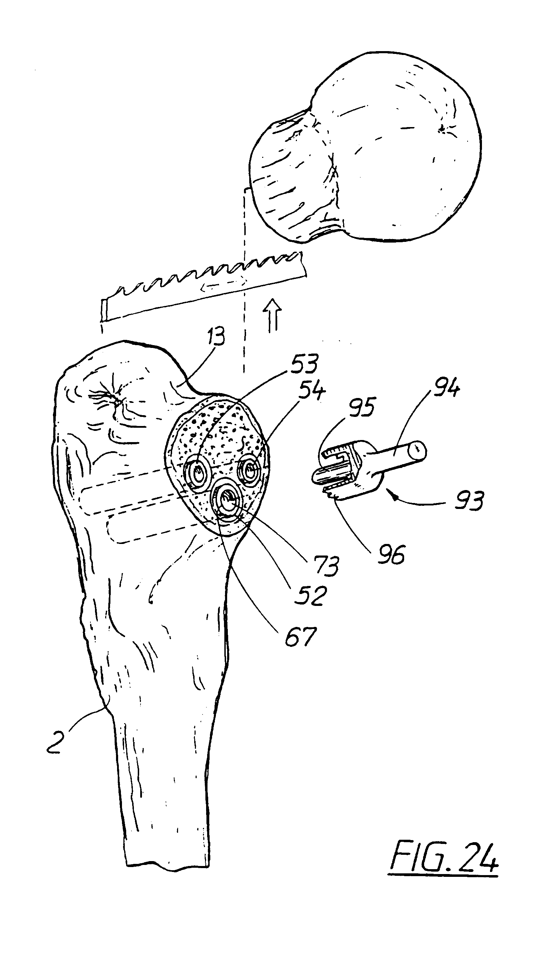

The design of the hip joint prosthesis in the second embodiment is shown most clearly in Figs. 20 and 21. As in the first embodiment, the hip joint prosthesis is made up of two joint units here: a first joint unit 45 anchored in the human femoral neck 13 and a second joint unit 46 anchored in the human pelvis 47. The joint unit anchored in the femur 2 has a joint head 48 anchored by means of an anchoring arrangement 49 which uses the same anchoring principle as that in the first embodiment, but which in other respects is of a different design. The joint head 48 also differs to the extent that there is no cavity for receiving a shaped part of the human femoral neck 13, but has instead an essentially plane bearing surface 50 for bearing against a plane bearing surface 51, made by cutting (see Fig. 24), in the human bone tissue. This bearing surface 51 in the bone tissue extends preferably at right angles to the longitudinal axes of the recessed channels 57. The anchoring arrangement 49 in the second embodiment does not have only one anchoring element, and one tightening element, but at least two. In the example shown there are three sleeve-shaped anchoring elements 52, 53, 54, and a number of tightening elements 55, 56 corresponding to the number of anchoring elements, i.e. one tightening element positioned in each anchoring element. The sleeve-shaped anchoring elements 52, 53, 54 are each introduced into a respective continuous channel 57 in the femur (see Fig. 23) and have an essentially cylindrical outer circumferential surface 58 which is provided with grooves which, in the example shown, are directed across the longitudinal direction of the anchoring elements and can be elongate linear grooves or, as in the example shown, can form a thread, i.e. extend in a spiral shape. The external diameters of the anchoring elements and the diameters of the recessed channels 57 are adapted in such a way that the anchoring elements completely fill the width of the channels and engage with the grooves in the bone tissue. However, the anchoring elements 52, 53, 54 have a section 59, directed towards the joint head 48, which does not make bone contact and expediently has a smooth cylindrical circumferential surface. Moreover, the anchoring elements fill the entire length of the recessed channels 57 up to the bearing surface 51 of the femoral neck. The anchoring elements 52 - 54 thus preferably extend at right angles to the bearing surface 50 of the joint head 48.

Fig. 21 shows the mutual positions and extents of the anchoring elements. It also shows that one anchoring element 52 expediently has a greater external and internal diameter than the two other anchoring elements 53, 54 and that the anchoring elements have a mutual positioning, viewed from their end, in which their mutually parallel longitudinal axes form, in a plane transverse thereto, the corners of an isosceles triangle with the larger anchoring element 52 positioned distal at the apex of the isosceles triangle, while the two smaller anchoring elements are positioned proximal and each form a corner of the triangle base, which is the shortest side of the triangle. The anchoring elements 52 - 54 are suitably made of a strong and tissue-compatible material, such as titanium, which permits osseointegration between the outer surface 58 and the bone tissue.

Fig. 20 shows that the joint head 48 has a number of essentially cylindrical flanges 60 projecting from the bearing surface 50 of the head, the number thereof corresponding to the number of anchoring elements. The flanges 60 extend rotationally symmetrical about the common longitudinal axes of the anchoring elements and tightening elements in their assembled state. Thus, in the example shown, three cylindrical flanges 60, 61 projecting from the bearing surface 50 of the joint head 48 are designed as integrated parts in the bearing surface of the joint head. The flanges 60, 61 have a cylindrical internal surface 62 for guiding and bearing against the external circumferential surface 58 of the associated anchoring element, more specifically at its narrowing, smooth part 59. The radius of curvature of this internal bearing surface 62 is adapted to the diameter of the smooth circumferential surface of the anchoring element in such a way that there is a good fit between the surfaces. Each flange also has an outer, essentially cylindrical or, for example, conical bearing surface 63 which is rotationally symmetrical about the anchoring element and tightening element and which is intended to bear against a recessed bearing surface 64 in the femoral neck. This bearing surface 64 is thus rotationally symmetrical about the associated recessed channel 57 in the femoral neck and forms a widened part of this at the end of the channel towards the joint head 48. The procedure for making this recess is explained in greater detail below with reference to Fig. 24.

The joint head also has a recess 65 for each tightening element 55, which recess 65 is shaped after the form of the end 66 of the tightening element and is, for example, essentially cylindrical and is either provided beforehand with internal threads or is formed for thread engagement with the end part of the tightening element, which end part is expediently threaded. Another technique for locking the tightening element in the joint head is of course conceivable. Thus, in the example shown, three mutually parallel recesses 65 are arranged, about which each of the three projecting bearing flanges 60, 61 are rotationally symmetrically arranged with an axis of symmetry which coincides with the axis of symmetry of the recesses 65. The recesses 65 are expediently dimensioned with such a depth that the tightening elements 55 do not reach the bottom of the recesses, but rather there is a certain amount of space for necessary tolerances and also for prestressing of the joint head 48 against the bearing surface 51 of the femoral neck. The load-absorbing surfaces, however, consist essentially of the annular end surfaces 67 of the tightening elements, against which end surfaces there bear corresponding surfaces 68 in the bottom of the cavity 69 designed as a hollow cylinder, except for the force absorption which is effected in the screw connection between the end 66 of the tightening element and the recess 65 in the joint head 48. The joint head 48 rests and is thus anchored in three take-up positions, which affords a stable force absorption as regards both tensile, bending and torsional forces. In a corresponding manner, there is a step-like surface at the transition between the recessed channel 57 in the femoral neck and its widened part with the bearing surface 64 in the bone tissue. This step-like surface 70 corresponds to the end surface 71 of each flange 60, which does not necessarily need to reach the bottom of the recess, but rather a space can exist for tolerances and prestressing. Thus, no forces are to be transmitted between these two surfaces 70, 71.

The tightening elements 55 are designed as screws which, however, only have to be threaded in their part situated outside the sleeve-shaped anchoring element. The main part 72 of the tightening element extends in the continuous and expediently cylindrical channel 73 formed by means of the sleeve shape of the anchoring element, with a preferably smooth internal circumferential wall 74. Correspondingly, the cylindrical part 72 of the tightening element in the channel is expediently smooth and dimensioned for good fitting in the anchoring element with small tolerances, so that the tightening element can, during assembly, be introduced into the anchoring sleeve with satisfactory guiding therein. Each of the tightening elements moreover has a head 75 which is expediently recessed in a conical countersink 76 in that end of the anchoring element distant from the joint head 48. For turning the tightening element 55 and, thus, screwing the latter into the joint head 48, a groove is arranged, for example for a hexagonal wrench. In order to improve the form connection to the outer surface of the leg, a flange 77 common to all three anchoring elements is connected firmly to the ends of the anchoring elements by means of these being threaded into holes in the flange. The flange 77 is shaped in such a way that it matches as well as possible the form of the femur 2. An important factor for the main parts of the anchoring arrangement, namely the anchoring elements and tightening elements, is that the anchoring elements should have such an extension and also be arranged in such a way that direct contact between tightening element and bone tissue is avoided, which is a precondition for implantation in two separate sequences. In the example shown in Fig. 20, the tightening elements are completely isolated from direct contact with bone tissue, and the anchoring is effected entirely via the anchoring elements.

The joint head 48 and the other joint unit 46, which is a joint socket unit, can be designed in different ways. In the example shown in Fig. 20, the joint head 48 is designed as an expediently solid partial sphere with a spherical surface 78. The joint head 48 is made, for example, of a strong and tissue-compatible material, such as titanium. The joint socket unit has a socket 79 which is likewise expediently made of titanium and which, by means of an anchoring arrangement 80, is anchored in the pelvis 47 by means of, for example, three anchoring elements 81 designed as sleeve-shaped elements, which are likewise expediently made of titanium and are introduced into previously formed channels in the hip bone. Grooves in the circumferential surface 82 ensure a secure engagement and osseointegration in the bone tissue. In this case too the anchoring elements are designed in such a way that direct contact is avoided between tightening elements 83 in the form of screws, which are introduced into the anchoring elements in a later operation sequence and screwed into the threaded cavity 84 of the anchoring elements. The screws 83 have heads 85 which are recessed in countersinks 86 in the socket 79 in such a way that they do not cross the concave surface 87 of the socket, which is made, for example, with a polymer lining or as a separate socket part secured in the joint socket in order to provide a good bearing against the joint head.

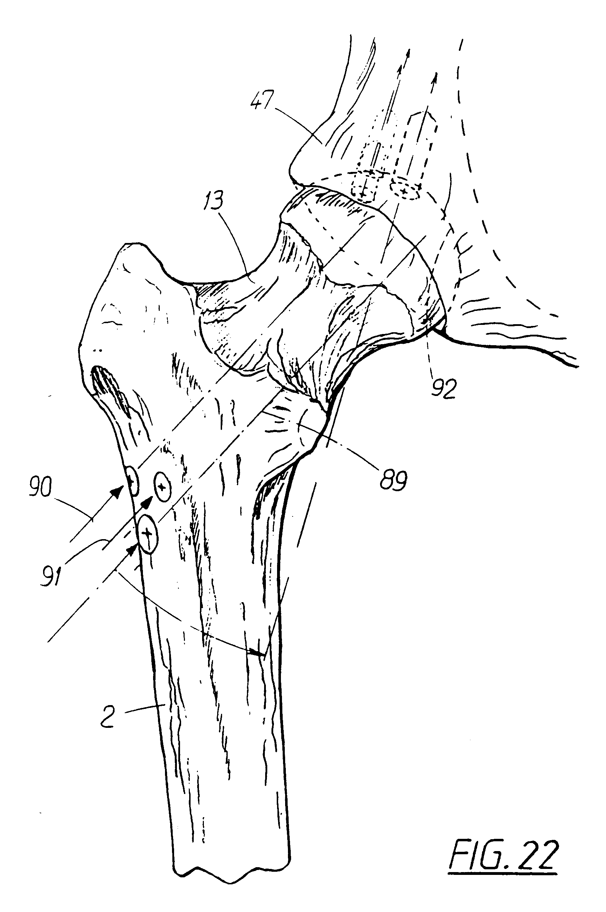

Implantation of the hip joint prosthesis shown in Fig. 20 is carried out in a way which shows similarities to the procedure in connection with implantation of the first embodiment. A drill guide (not shown) is introduced under TV fluoroscopy through the upper part of the side of the femur, up through the lower part of the femoral neck and penetrating the joint head in a direction indicated by the dot-and-dash line 89 in Fig. 22. The first of the channels described above in the femur 2 is then made by guiding a drill guide. Thereafter, the two other channels are made in a cutting direction indicated by the arrows 90, 91, i.e. parallel to the direction of the first channel. With continued TV fluoroscopy, the hip is put into a position in which the first drilled channel points centrally up into the pelvis 47. Through the distal channel, i.e. the said first channel, a hole is drilled and threaded into the acetabulum 92, i.e. into the pelvis. One of the anchoring elements 81 for the socket unit 46 is then threaded into the acetabulum. In a corresponding way, channels are made in the acetabulum for other anchoring elements by suitable adjustment and fixing of the joint.

After the continuous channels have been made in the femoral neck, the channels are threaded and the three anchoring elements 52, 53, 54 are introduced into the associated channels. Thereafter, the first operation sequence is finished, and the implant is allowed to set.

The second operation sequence is initiated by opening and exposing the hip joint, after which the femoral neck is sawn off at the level of the previously introduced anchoring elements 52, 53, 54, as shown in Fig. 24. This affords the plane bearing surface 51 of bone tissue in the femoral neck. As is also shown in Fig. 24, a cutting instrument 93 in the form of a so-called trepan is then used to cut the bone tissue in order to shape the recesses which are intended to take up the collar-like flanges 60 around the ends 59 of the anchoring elements. This cutting is carried out with high precision by virtue of the fact that the implanted anchoring elements 52, 53, 54, more specifically their continuous cylindrical channel 73, form an axle guide for the cutting tool. The latter is in fact designed with, in addition to a drive shaft 94 connected to a drive motor, a forward extension in the form of a guide axle 95 which expediently is essentially cylindrical and has a diameter which, with suitable tolerance, corresponds to the internal diameter of the anchoring elements. The cutting instrument moreover has a circular saw 96 which is arranged rotationally symmetrical about the guide axle 95, produces a cylindrical cutting and is dimensioned in such a way that the recess in the bone tissue takes up the collar-like flanges 60 with good fitting. In the example shown, two different dimensions of the cutting instrument are thus required for matching with the two different dimensions of the anchoring elements and flanges. The cutting instrument is advantageously adapted so that a desired depth has been achieved when the instrument reaches the end surfaces 67 of the anchoring elements.

The socket unit 46 is then secured in its anchoring elements 81 by means of tightening elements 83. It should be ensured that the bearing surface 51 of the femoral neck is carefully adapted to the end surfaces 67 of the anchoring elements, in which respect, for example, the bearing surface 51 can be situated slightly in front of the bearing surfaces 67 so that a certain prestressing can be achieved. The joint head 48 is then secured on the femoral neck by means of the flanges 60 being introduced into their recesses and the tightening elements 55 being introduced into the anchoring elements and their ends screwed into the joint head until the end surfaces 67 of the anchoring elements reach the surfaces 68 of the joint head. In this respect, the plane bearing surface 50 of the joint head 48 is advantageously pressed against the plane, cut bearing surface 51 of the femoral neck with a certain prestressing. The hip joint is then reset and the wound is closed, after which the operation is complete.

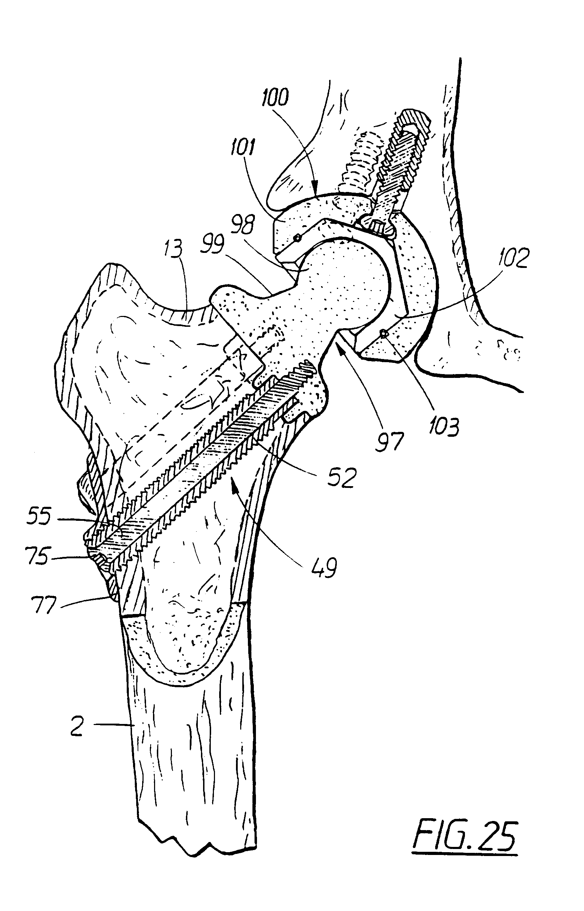

Fig. 25 shows an example of a further embodiment in which the joint head, here designated by 97, has another shape with a more pronounced ball 98 and a neck part 99. Otherwise, the prosthesis is anchored in the femur in the same way as in the embodiment described above. Furthermore, the socket unit, here designated by 100, is divided into two sections: on the one hand a base socket 101, which is anchored in the manner described above for the second embodiment, and an inner socket 102, which can be made of a wear-resistant low-friction material. The inner socket 102 is, for example, secured in the base socket by means of a snap lock 103, locking ring or the like.

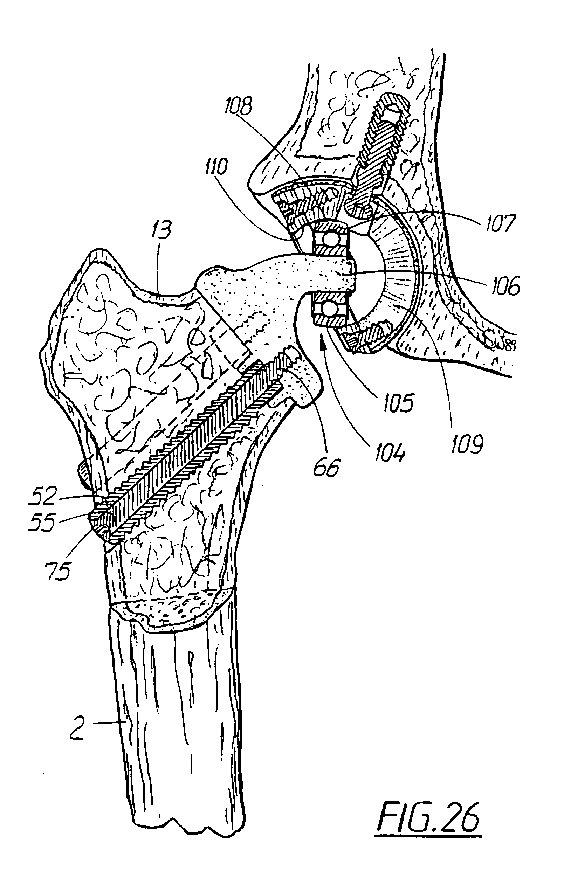

The embodiment shown in Fig. 26 has a joint head, here designated by 104, of a completely different type. Here, the joint head is designed as a ball bearing 105, mounted around an axle 106 formed in the joint head, which thus permits rotation of an outer bearing part 107 about the said axle, and a slide bearing in its outer periphery 108 which has the form of a spherical zone, which is adapted to the joint surface of the joint socket 109. In this case the joint socket is provided with an annular element 110 which gives the socket a spherical surface, which exceeds a semisphere, by which means the joint head in the form of the ball bearing 105 cannot be removed from its articulating connection with the socket unit.

The invention is not limited to the exemplary embodiments described above and shown in the drawings, but can be varied within the scope of the subsequent patent claims. For example, it is conceivable for the prestressable anchoring arrangement for the joint head to have another detail design. For example, it is conceivable for the pin 14 to have a length which corresponds essentially to the length of the anchoring element 6 so that the screw 7 projects, in the assembled state, slightly outside the flange 20 of the anchoring element and is prestressed with a nut instead of the screw bushing 8. In this case, the nut can be expediently countersunk or encapsulated. Alternatively, the screw 7 can be replaced by a sleeve which forms the pin 14 and has a bore with an internal thread, in which connection the screw bushing 8 is replaced by a screw.

The socket unit can have a completely different design from the unit shown.

The joint head and its surfaces bearing against the bone tissue can also be designed in several different ways. A non-plane bearing surface between the joint head and femoral neck is also conceivable in an embodiment with three anchoring elements. Even if the anchoring arrangement is prestressable, it is not necessary per se to use the prestressing possibility in each application. The expression "grooved outer surface" also includes "threaded outer surface". In the embodiment with three anchoring elements it is not necessary to choose the configuration of an isosceles triangle, and another triangle with different sides is conceivable.

## Claims

1. Hip joint prosthesis for permanent anchoring in the human hip joint, which prosthesis consists of a first joint unit (1; 45) designed to be anchored in the neck of a human femur (2) and a second joint unit (3; 46) designed to be anchored in the human pelvis (4; 47), in which respect the first joint unit comprises a joint head (5; 48; 97; 104) and an anchoring arrangement (6, 7, 8; 49) for anchoring the joint head in the femur, and the second joint unit comprises a socket (28; 79; 102; 109) with a concave joint surface (31; 87; 110) shaped and dimensioned to bear against and to articulate under sliding contact with the joint head, and an anchoring arrangement (34; 80) for anchoring the socket in a cavity (35) in the pelvis, the anchoring arrangement in the femur comprising at least one sleeve-shaped anchoring element (6; 52, 53, 54) and at least one tightening element (7, 8; 14; 55) connected to the joint head in order to create, by engaging with the anchoring element, a prestressable anchoring of the joint head with the tightening element introduced into the sleeve-shaped anchoring element, the tightening element comprising a head (24; 75) and being designed to be axially introduced into the associated sleeve-shaped anchoring element and connected to the joint head, characterized in that the sleeve-shaped anchoring element (6; 52, 53, 54) has a grooved outer surface (16; 58) and is designed to be separately implanted in a channel (17) passing through the femoral neck (13) and to extend essentially over the entire length thereof in order to form an anchorage in the femur via the said outer surface and to form a guide for the tightening element (7, 8, 14; 55) of the first joint unit (1; 45) and an axle guide for a cutting instrument rotatable about an axle (45; 95) for shaping at least one bearing surface (11; 64) of the femoral neck, extending rotationally symmetrically about the longitudinal axle of the said tightening element, to the shape of at least one corresponding bearing surface of the joint head (5; 48; 97; 104) extending rotationally symmetrically about the attachment of the said tightening element in the joint head.

2. Hip joint prosthesis according to Patent Claim 1, in which the joint head (5) consists of a ball (5) with a convex joint surface (9), characterized in that the ball (5) has a cavity (10) designed to be arranged on the end of the human femoral neck (13), which is shaped for fitting in the said cavity, and a shaft (14) extending essentially centrally from the cavity, in that the anchoring arrangement (6, 7, 8) for the ball comprises a single sleeve-shaped anchoring element (6) and a joining piece connected to the said shaft in the form of a prestressing device (8) in order to create, by engaging with the anchoring element, a prestressing of the ball with the shaft introduced into the sleeve-shaped anchoring element.

3. Hip joint prosthesis according to Patent Claim 2, characterized in that the prestressing device comprises a prestressing sleeve (8) which is designed to be introduced into the sleeve-shaped anchoring element (6) and is coupled to the shaft (14) of the ball (5) by means of the said joining piece which constitutes a screw connection.

4. Hip Joint prosthesis according to Patent Claim 3, characterized in that the said screw connection consists of a threaded part (7) in the shaft (14) of the ball (5) and a threaded part (23) in the prestressing sleeve (8), which threaded parts are intended to be in thread engagement with each other at variable relative positions, by means of which the ball can be prestressed against the femoral neck (13).

5. Hip joint prosthesis according to Patent Claim 4, characterized in that the anchoring element (6) for the ball (5) has a flange (20) which, when the prosthesis is in the assembled state, is situated at the end distant from the ball, and in that the prestressing sleeve (8) has a head (24) designed to bear against the flange.

6. Hip joint prosthesis according to any one of the preceding Patent Claims, characterized in that the sleeve-shaped anchoring element (6) has a continuous channel (18) which forms the said axle guide for the said cutting instrument, designed to shape the femoral neck (13) to the shape of the cavity (10) of the ball (5).

7. Hip Joint prosthesis according to any one of the preceding Patent Claims, characterized in that the cavity (10) of the ball (5) is essentially cylindrical and profiled in the cylinder surface.

8. Hip joint prosthesis according to Patent Claim 1, characterized in that the anchoring arrangement (49) consists of at least two sleeve-shaped anchoring elements (52 - 54) arranged essentially parallel to each other and each in a channel in the femoral neck (13), and at least two tightening elements (55) extending in each of the anchoring elements.

9. Hip joint prosthesis according to Patent Claim 8, characterized in that the anchoring elements and the tightening elements (55) are three in number and are each arranged in a longitudinal axis cutting through the joint head (48) eccentrically and forming, in a plane at right angles to the longitudinal axes, the corners of a triangle.

10. Hip joint prosthesis according to Patent Claim 9, characterized in that the one anchoring element (52) and the associated tightening element (55) have a greater diameter than the other anchoring elements (53, 54) and associated tightening elements (55).

11. Hip joint prosthesis according to Patent Claim 10, characterized in that the said triangle is isosceles, the anchoring element (52) of greater diameter being situated at the apex of the triangle.

12. Hip joint prosthesis according to Patent Claim 8, characterized in that the joint head (48) has, on the one hand, an essentially plane bearing surface (50) directed towards the femoral neck (13) and designed to bear against a plane bearing surface (51) of the femoral neck formed by cutting and, on the other hand, a number of flanges (60, 61) corresponding to the number of anchoring elements (52 - 55), which flanges (60, 61) project from the bearing surface of the joint head and are designed to surround one end part of the anchoring elements and to form, with their outside, the said rotationally symmetrical bearing surface (70) of the joint head for bearing against the said rotationally symmetrical bearing surface (71) formed in the femoral neck by means of the cutting instrument (93).

13. Hip joint prosthesis according to Patent Claim 12, characterized in that the said flanges (60, 61) have a stop surface (68) designed to bear against and to transfer to the end face (67) of the anchoring element (52 - 55) forces acting on the joint head (48).

14. Hip joint prosthesis according to Patent Claim 13, characterized in that each tightening element (55) consists of a screw (55) with a head (75) designed to bear against that end (76) of the anchoring element (52, 53, 54) distant from the joint head (48) and designed, with a threaded part (66), to be in thread engagement with the joint head in a recess (65) therein.

15. Hip joint prosthesis according to Patent Claim 1, characterized in that the said anchoring arrangement (34) for the joint socket (28) comprises sleeve-shaped anchoring elements (36) which are implanted separately in the pelvis and into which a number of screws (40) introduced through holes (41) in the socket are intended to be screwed.

16. Hip joint prosthesis according to Patent Claim 15, characterized in that the joint socket (3) consists of, on the one hand, a load-absorbing base socket (28) which is provided with the said holes (41) and, on the other hand, a joint socket (29) which is secured in the base socket by means of positive locking (33, 34).

## Patentansprüche

1. Hüftgelenkprothese zur permanenten Verankerung in dem menschlichen Hüftgelenk, bestehend aus einer ersten Gelenkeinheit (1; 45), die zur Verankerung in dem Hals eines menschlichen Femurs (2) ausgebildet ist, und aus einer zweiten Gelenkeinheit (3; 46), die zur Verankerung in der menschlichen Pelvis (4; 47) ausgebildet ist, wobei die erste Gelenkeinheit einen Gelenkkopf (5; 48; 97; 104) und eine Verankerungsanordnung (6, 7, 8; 49) zur Verankerung des Gelenkkopfes in dem Femur aufweist und wobei die zweite Gelenkeinheit einen Sockel (28; 79; 102; 109) mit einer konkaven Gelenkfläche (31; 87; 110), die zur Lagerung an und zur Bewegung unter gleitendem Kontakt mit dem Gelenkkopf geformt und dimensioniert ist, und eine Verankerungsanordnung (34; 80) zur Verankerung des Sockels in einer Gelenkhöhle (35) in der Pelvis besitzt, wobei die Verankerungsanordnung in dem Femur wenigstens ein hülsenförmiges Verankerungselement (6; 52, 53, 54) und wenigstens ein Festspannelement (7, 8; 14,; 55) aufweist, das zur Schaffung einer vorspannbaren Verankerung des Gelenkkopfes mit dem in das hülsenförmige Verankerungselement eingeführten Festspannelement mittels Eingriff mit dem Verankerungselement mit dem Gelenkkopf verbunden ist, wobei das Festspannelement einen Kopf (24; 75) besitzt, zur axialen Einführung in das zugeordnete hülsenförmige Verankerungselement ausgebildet und mit dem Gelenkkopf verbunden ist,
dadurch **gekennzeichnet**, daß das hülsenförmige Verankerungselement (6; 52, 53, 54) eine genutete Außenfläche (16; 58) besitzt und separat in einen Kanal (17), der durch den Femurhals (13) tritt, implantierbar ist und sich im wesentlichen über dessen gesamte Länge zur Bildung einer Verankerung in dem Femur über die Außenfläche und zur Bildung einer Führung für das Festspannelement (7, 8; 14; 55) der ersten Gelenkeinheit (1; 45) sowie einer Achsenführung für ein Bearbeitungsinstrument erstreckt, das um eine Achse (45; 95) zur Gestaltung wenigstens einer Lagerfläche (11; 64) des Femurhalses, die sich rotationssymmetrisch um die Längsachse des Festspannelementes erstreckt, in die Form wenigstens einer korrespondierenden Lagerfläche des Gelenkkopfes (5; 48; 97; 104) drehbar ist, die sich rotationssymmetrisch um die Befestigung des Festspannelements in dem Gelenkkopf erstreckt.

2. Hüftgelenkprothese nach Anspruch 1, bei der der Gelenkkopf (5) aus einer Kugel (5) mit einer konvexen Gelenkfläche (9) besteht, dadurch **gekennzeichnet**, daß die Kugel (5) einen Hohlraum (10), der zur Anordnung auf dem Ende des menschlichen Femurhalses (13) ausgebildet ist, welcher in den Hohlraum passend gestaltet ist, und einen Schaft (14) besitzt, der sich im wesentlichen zentrisch von dem Hohlraum erstreckt, daß die Verankerungsanordnung (6, 7, 8) für die Kugel ein einzelnes hülsenförmiges Verankerungselement (6) und ein mit dem Schaft verbundenes Anschlußteil in Form einer Vorspanneinrichtung (8) zur Schaffung eines Vorspannens der Kugel mit dem in das hülsenförmige Verankerungselement eingeführten Schaft durch Eingriff mit dem Verankerungselement besitzt.

3. Hüftgelenkprothese nach Anspruch 2, dadurch **gekennzeichnet**, daß die Vorspanneinrichtung eine Vorspannhülse (8) besitzt, die in das hülsenförmige Verankerungselement (6) einführbar ist und mit dem Schaft (14) der Kugel (5) mittels des Anschlußstückes verbunden ist, das eine Schraubverbindung bildet.

4. Hüftgelenkprothese nach Anspruch 3, dadurch **gekennzeichnet**, daß die Schraubverbindung aus einem Gewindeabschnitt (7) in dem Schaft (14) der Kugel (5) und einem Gewindeabschnitt (23) in der Vorspannhülse (8) besteht, wobei die Gewindeabschnitte für einen Gewindeeingriff miteinander in variierbaren, relativen Stellungen vorgesehen sind, wodurch die Kugel gegen den Femurhals (13) vorspannbar ist.

5. Hüftgelenkprothese nach Anspruch 4, dadurch **gekennzeichnet**, daß das Verankerungselement (6) für die Kugel (5) einen Flansch (20) besitzt, der sich dann, wenn sich die Prothese im zusammengebauten Zustand befindet, an dem von der Kugel entfernten Ende befindet, und daß die Vorspannhülse (8) einen Kopf (24) besitzt, der für das Anliegen gegen den Flansch ausgebildet ist.

6. Hüftgelenkprothese nach einem der vorangehenden Ansprüche, dadurch **gekennzeichnet**, daß das hülsenförmige Verankerungselement (6) einen kontinuierlichen Kanal (18) besitzt, der die Achsenführung für das Bearbeitungsinstrument bildet, welches zur Gestaltung des Femurhalses (13) in die Form des Hohlraums (10) der Kugel (5) vorgesehen ist.

7. Hüftgelenkprothese nach einem der vorangehenden Ansprüche, dadurch **gekennzeichnet**, daß der Hohlraum (10) der Kugel (5) im wesentlichen zylindrisch und in der Zylinderfläche profiliert ist.

8. Hüftgelenkprothese nach Anspruch 1, dadurch **gekennzeichnet**, daß die Verankerungsanordnung (49) aus wenigstens zwei hülsenförmigen Verankerungselementen (52-54), die im wesentlichen parallel zueinander und jeweis in einem Kanal in dem Femurhals (13) angeordnet sind, und aus wenigstens zwei Festspannelementen (55) besteht, die sich in jedem der Verankerungselemente erstrecken.

9. Hüftgelenkprothese nach Anspruch 8, dadurch **gekennzeichnet**, daß die Verankerungselemente und die Festspannelemente (55) dreifach vorhanden und jeweils in einer Längsachse angeordnet sind, die exzentrisch den Gelenkkopf (48) durchschneidet und die in einer Ebene rechtwinklig zu den Längsachsen die Ecken eines Dreiecks bilden.

10. Hüftgelenkprothese nach Anspruch 9, dadurch **gekennzeichnet**, daß ein Verankerungselement (52) und das zugeordnete Festspannelement (55) einen größeren Durchmesser als die anderen Verankerungselemente (53, 54) und deren zugeordnetes Festspannelement (55) besitzt.

11. Hüftgelenkprothese nach Anspruch 10, dadurch **gekennzeichnet**, daß das Dreieck gleichschenklig ist, wobei das Verankerungselement (52) mit größerem Durchmesser an der Spitze des Dreiecks liegt.

12. Hüftgelenkprothese nach Anspruch 8, dadurch **gekennzeichnet**, daß der Gelenkkopf (48) einerseits eine im wesentlichen ebene Lagerfläche (50), die auf den Femurhals (13) gerichtet ist und für das Anliegen an einer ebenen Lagerfläche (51) des Femurhalses, die durch Bearbeitung gebildet ist, und andererseits eine Zahl von Flanschen (60, 61) in entsprechender Zahl zu den Verankerungselementen (52-55) aufweist, wobei die Flansche (60, 61) von der Lagerfläche des Gelenkkopfes vorstehen und derart ausgebildet sind, daß sie einen Endabschnitt der Verankerungselemente umgeben und mit deren Außenseite die rotationssymmetrische Lagerfläche (70) des Gelenkkopfes zur Lagerung an der rotationssymmetrischen Lagerfläche (71) bilden, die an dem Femurhals mittels des Bearbeitungsinstrumentes (93) geformt ist.

13. Hüftgelenkprothese nach Anspruch 12, dadurch **gekennzeichnet**, daß die Flansche (60, 61) eine Anschlagfläche (68) besitzen, die zur Anlage gegen die Endfläche (67) des Verankerungselements (52-55) und zur Übertragung von Kräften ausgebildet ist, die auf den Gelenkkopf (48) wirken.

14. Hüftgelenkprothese nach Anspruch 13, dadurch **gekennzeichnet**, daß jedes Festspannelement (55) aus einer Schraube (55) mit einem Kopf (75) besteht, der für die Anlage gegen das Ende (76) des Verankerungselements (52, 53, 54) im Abstand von dem Gelenkkopf (48) und mit einem Gewindeabschnitt (66) für einen Gewindeeingriff mit dem Gelenkkopf in einer Ausnehmung (65) desselben ausgebildet ist.

15. Hüftgelenkprothese nach Anspruch 1, dadurch **gekennzeichnet**, daß die Verankerungsanordnung (34) für den Gelenksockel (28) hülsenförmige Verankerungselemente (36) besitzt, die separat in der Pelvis implantiert sind und in die eine Zahl von Schrauben (40), die durch Löcher (41) in dem Sockel eingeführt sind, einschraubbar sind.

16. Hüftgelenkprothese nach Anspruch 15, dadurch **gekennzeichnet**, daß der Gelenksockel (3) einerseits aus einem lastabsorbierenden Grundsockel (28), der mit den Löchern (41) versehen ist, und andererseits aus einem Gelenksockel (29) besteht, der in dem Grundsockel durch Formschluß (33, 34) befestigt ist.

## Revendications

1. Prothèse d'articulation de la hanche destinée à assurer un ancrage permanent dans une articulation de hanche humaine, la prothèse étant constituée d'un premier élément d'articulation (1 ; 45) destiné à être ancré dans le col d'un fémur humain (2) et d'un second élément d'articulation (3 ; 46) destiné à être ancré dans le bassin humain (4 ; 47), le premier élément d'articulation comporte une tête d'articulation (5 ; 48 ; 97 ; 104) et un ensemble d'ancrage (6, 7, 8 ; 49) destiné à ancrer la tête d'articulation dans le fémur, et le second élément d'articulation comprend une douille (28 ; 79 ; 102 ; 109) ayant une surface concave d'articulation (31 ; 87 ; 110) dont la configuration et les dimensions sont telles qu'elle peut être en appui contre la tête d'articulation et peut s'articuler par contact glissant avec cette tête, et un ensemble d'ancrage (34 ; 80) destiné à ancrer la douille dans une cavité (35) du bassin, l'ensemble d'ancrage du fémur comprenant au moins un élément d'ancrage en forme de manchon (6 ; 52, 53, 54) et au moins un élément de serrage (7, 8 ; 14 ; 55) raccordé à la tête d'articulation afin qu'il crée, par coopération avec l'élément d'ancrage, un ancrage de tête d'articulation qui peut être mis sous une contrainte préalable, l'élément de serrage étant introduit dans l'élément d'ancrage en forme de manchon, l'élément d'ancrage comprenant une tête (24 ; 75) et étant destiné à être introduit axialement dans l'élément associé d'ancrage en forme de manchon et raccordé à la tête d'articulation, caractérisé en ce que l'élément d'ancrage en forme de manchon (6 ; 52, 53, 54) a une surface externe (16 ; 58) à gorges et est réalisé afin qu'il soit implanté séparément dans un canal (17) passant à travers le col du fémur (13) et soit disposé pratiquement sur toute la longueur de celui-ci pour la formation d'un ancrage dans le fémur par l'intermédiaire de la surface externe, et forme un guide pour l'élément de fixation (7, 8, 14 ; 55) du premier élément d'articulation (1 ; 45) et un guide d'arbre pour un instrument de coupe qui peut tourner autour d'un arbre (45 ; 95) afin qu'il mette en forme au moins une surface d'appui (11 ; 64) du col du fémur, disposée symétriquement en rotation autour de l'arbre longitudinal de l'élément de serrage, à la configuration d'au moins une surface correspondante d'appui de la tête d'articulation (5 ; 48 ; 97 ; 104) disposée symétriquement en rotation autour de la fixation de l'élément de serrage à la tête d'articulation.

2. Prothèse d'articulation de la hanche selon la revendication 1, dans laquelle la tête (5) d'articulation est constituée d'une rotule (5) ayant une surface convexe d'articulation (9), caractérisée en ce que la rotule (5) a une cavité (10) destinée à être placée à l'extrémité du col du fémur humain (13), dont la configuration permet son logement dans la cavité, et un arbre (14) disposé pratiquement au centre depuis la cavité, en ce que l'ensemble d'ancrage (6, 7, 8) de la rotule comprend un seul élément d'ancrage en forme de manchon (6) et une pièce d'articulation raccordée à l'arbre sous forme d'un dispositif (8) d'application d'une contrainte préalable de manière que, par coopération avec l'ensemble d'ancrage, une contrainte préalable de la rotule soit créée avec l'arbre introduit dans l'élément d'ancrage en forme de manchon.

3. Prothèse d'articulation de la hanche selon la revendication 2, caractérisée en ce que le dispositif de mise sous contrainte préalable comporte un manchon (8) de mise sous contrainte préalable qui est destiné à être introduit dans l'élément d'ancrage en forme de manchon (6) et qui est couplé à l'arbre (14) de la rotule (5) par la pièce d'articulation qui constitue un raccord vissé.

4. Prothèse d'articulation de la hanche selon la revendication 3, caractérisée en ce que le raccord vissé comprend une partie filetée (7) formée dans l'arbre (14) de la rotule (5) et une partie taraudée (23) formée dans le manchon de mise sous contrainte préalable (8), les parties filetée et taraudée étant destinées à coopérer par vissage en des positions relatives variables, si bien que la rotule peut être mise à une contrainte préalable contre le col du fémur (13).

5. Prothèse d'articulation de la hanche selon la revendication 4, caractérisée en ce que l'élément d'ancrage (6) de la rotule (5) a un flasque (20) qui, lorsque la prothèse est à l'état assemblé, se trouve à l'extrémité distante de la rotule, et en ce que le manchon (8) de mise sous contrainte préalable possède une tête (24) destinée à être en appui contre le flasque.

6. Prothèse d'articulation de la hanche selon l'une quelconque des revendications précédentes, caractérisée en ce que l'élément d'ancrage (6) en forme de manchon a un canal continu (18) qui forme le guide d'arbre de l'instrument de coupe, réalisé de manière qu'il mette le col du fémur (13) à la configuration de la cavité (10) de la rotule (5).

7. Prothèse d'articulation de la hanche selon l'une quelconque des revendications précédentes, caractérisée en ce que la cavité (10) de la rotule (5) a une forme essentiellement cylindrique et est profilée à la surface du cylindre.

8. Prothèse d'articulation de la hanche selon la revendication 1, caractérisée en ce que l'ensemble d'ancrage (49) est constitué d'au moins deux éléments d'ancrage en forme de manchons (52, 54) qui sont pratiquement parallèles l'un à l'autre, placés chacun dans un canal du col du fémur (13), et d'au moins deux éléments de serrage (55) disposés dans chacun des éléments d'ancrage.

9. Prothèse d'articulation de la hanche selon la revendication 8, caractérisée en ce que les éléments d'ancrage et les éléments de serrage (55) sont au nombre de trois, ils sont disposés chacun suivant un axe longitudinal qui recoupe la tête (48) d'articulation de manière excentrique, et ils forment, dans un plan perpendiculaire aux axes longitudinaux, les coins d'un triangle.

10. Prothèse d'articulation de la hanche selon la revendication 9, caractérisée en ce que le premier élément d'ancrage (52) et l'élément associé de serrage (55) ont un diamètre supérieur à celui des autres éléments d'ancrage (53, 54) et des éléments associés de serrage (55).

11. Prothèse d'articulation de la hanche selon la revendication 10, caractérisée en ce que ledit triangle est isocèle, l'élément d'ancrage (52) de plus grand diamètre étant placé au sommet de l'axe de symétrie du triangle.

12. Prothèse d'articulation de la hanche selon la revendication 8, caractérisée en ce que la tête d'articulation (48) possède, d'une part, une surface essentiellement plane d'appui (50) dirigée vers le col du fémur (13) et destinée à être en appui contre une surface plane d'appui (51) du col du fémur formée par découpe et d'autre part un certain nombre de flasques (60, 61) correspondant au nombre d'éléments d'ancrage (52-55), les flasques (60, 61) dépassant de la surface d'appui de la tête d'articulation et étant réalisés afin qu'ils entourent une partie d'extrémité des éléments d'ancrage et forment, avec l'extérieur, la surface d'appui à symétrie de rotation (70) de la tête d'articulation destinée à être en appui contre la surface d'appui (71) à symétrie de rotation formée dans le col du fémur par l'instrument de coupe (93).

13. Prothèse d'articulation de la hanche selon la revendication 12, caractérisée en ce que les flasques (60, 61) ont une surface d'arrêt (68) destinée à encaisser et transférer, à la face d'extrémité (67) de l'élément d'ancrage (52-55), les forces agissant sur la tête (48) d'articulation.

14. Prothèse d'articulation de la hanche selon la revendication 13, caractérisée en ce que chaque élément de serrage (55) est formé d'une vis (55) ayant une tête (75) destinée à être en appui contre l'extrémité (76) de l'élément d'ancrage (52, 53, 54) qui est distante de la tête d'articulation (48) et qui est destinée à coopérer, par une partie filetée (66) par vissage avec une cavité (65) formée dans la tête d'articulation.

15. Prothèse d'articulation de la hanche selon la revendication 1, caractérisée en ce que l'ensemble d'ancrage (34) de la douille (28) d'articulation comprend des éléments d'ancrage (36) en forme de manchons qui sont implantés séparément dans le bassin et dans lesquels un certain nombre de vis (40) introduites par des trous (41) de la douille sont destinées à être vissées.

16. Prothèse d'articulation de la hanche selon la revendication 15, caractérisée en ce que la douille (3) d'articulation est constituée, d'une part, d'une douille (28) de base qui absorbe les forces et qui a lesdits trous (41) et, d'autre part, d'une douille d'articulation (29) qui est fixée dans la douille de base par blocage positif (33, 34).
